# EUROPÄISCHE PATENTANMELDUNG

(11) **EP 4 108 650 A1**
(43) Veröffentlichungstag der Anmeldung: **28.12.2022**
(21) Anmeldenummer: 22160892.0
(22) Anmeldetag: 08.03.2022
(51) Int. Cl.: C07C 7/10, C07C 15/06, C07C 29/80, C07C 29/86, C07C 31/10

(54) **VERFAHREN ZUM AUFTRENNEN AZEOTROPER GEMISCHE**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Die vorliegende Erfindung bezieht sich auf ein Verfahren zum Auftrennen eines azeotropen Gemisches (A) umfassend Toluol und n-Propanol, dadurch gekennzeichnet, dass

(i) dem azeotropen Gemisch (A) Ethylenglycol oder Wasser zugesetzt wird, bis eine Mischungslücke erreicht ist, und

(ii) die entstehende Toluol-reiche Phase (B) von der 1-Propanol-haltigen Phase (C) mechanisch entfernt wird.

## Beschreibung

### Hintergrund

Toluol und 1-Propanol sind weit verbreitete industrielle Lösungsmittel in der Chemie. Bei der Herstellung eines Stoffes, z.B. eines Wirkstoffzwischenprodukts, bei dem optional auch Wasser als Nebenprodukt gebildet wird, muss häufig ein Lösungsmittelaustausch von 1-Propanol (CAS-Nr.: 71-23-8) (auch n-Propanol) zu Toluol (CAS-Nr.: 108-88-3) oder umgekehrt durchgeführt werden.

### Beschreibung des Standes der Technik

Sollte bei einer Reaktion Wasser als Nebenprodukt entstehen, muss das ternäre, homogene, azeotrope Gemisch aus 1-Propanol, Toluol und Wasser dabei destilliert werden, um einen erfolgreichen Lösungsmittelaustausch von einem System auf 1-Propanolbasis zu einem System auf Toluolbasis sicherzustellen. Dies beinhaltet generell nicht nur die Erzeugung eines großen Abfallstroms, der verbrannt werden muss, sondern auch große Mengen an Toluol, um einen effektiven Lösungsmittelaustausch sicherzustellen. Das Durchführen des Lösungsmittelaustauschs bei höheren Drücken und daher höheren Temperaturen um das Azeotrop günstig in einen 1-Propanol-reichen Strom zu verschieben, wäre auch aufgrund der Erzeugung unerwünschter Nebenkomponenten nicht geeignet.

CN106336337 betrifft ein Verfahren zum Trennen von Toluol und n-Propanol (1-Propanol) mit vollständiger wärmeintegrierter Druckwechseldestillation. Das Verfahren nutzt die Druckempfindlichkeitseigenschaften des aus Toluol und n-Propanol gebildeten Azeotrops und verwendet Doppeltürme mit hohem und niedrigem Druck, um eine möglichst wirksame Trennung zu erreichen.

DD 000000225049 beschreibt ein Verfahren zur Entfernung von geringen Mengen Toluol aus einem Lösungsmittelgemisch aus Methanol, Ethanol und Wasser. Dabei wird eine Mischungslücke in diesem Gemisch durch Zugabe von Wasser ausgenutzt und es erfolgt eine mechanische Trennung des Toluols von der wässrigen Phase (Methanol, Ethanol und Wasser).

Asma Iqbal und Syed Akhlaq Ahmad (Separating Iso-Propanol-Toluole mixture by azeotropic distillation; AIP Conference Proceedings 1953, 030016 (2018)) beschreiben ein Verfahren, das geeignet ist, die azeotrope Mischung von Iso-Propanol und Toluol durch Zugabe von Aceton durch verschiedene Destillationsschritte zu ermöglichen. Dieses Verfahren benötigt jedoch große Mengen Aceton, die zusätzlich entsorgt werden müssen.

### Zusammenfassung der Erfindung

Ein Aspekt der vorliegenden Erfindung bezieht sich auf ein Verfahren zur Auftrennung eines azeotropen Gemisches (A) umfassend Toluol und 1-Propanol, bevorzugt der Abtrennung von Toluol aus einem azeotropen Gemisch (A), wobei der Anteil an Toluol in dem Gemisch im Bereich von 55% (w/w) bis 80% (w/w) bezogen auf das Gesamtgewicht des azeotropen Gemisches (A) liegt und der Anteil an 1-Propanol im Bereich von 20% (w/w) bis 45% (w/w) bezogen auf das Gesamtgewicht des azeotropen Gemisches (A) liegt und der Anteil von weiteren Bestandteile des azeotropen Gemisches (A) im Bereich von 0% (w/w) bis 10% (w/w) bezogen auf das Gesamtgewicht des azeotropen Gemisches (A) liegt, wobei die Summe aus dem Toluol-Anteil, dem 1-Propanol-Anteil und dem Anteil von weiteren Bestandteilen zusammen immer 100 ergeben müssen, dadurch gekennzeichnet, dass
(i) dem azeotropen Gemisch (A) Ethylenglycol oder Wasser zugesetzt wird, bis eine Mischungslücke erreicht ist, und
(ii) die entstehende Toluol-reiche Phase (B) von der 1-Propanol-haltigen Phase (C) mechanisch entfernt wird.

In einer Ausführungsform 1 ist das Verfahren gemäß dem Aspekt dadurch gekennzeichnet, dass dem azeotropen Gemisch (A) in Schritt (i) Ethylenglykol zugesetzt wird.

In einer weiteren Ausführungsform 2 ist das Verfahren gemäß dem Aspekt und/oder der Ausführungsform 1 dadurch gekennzeichnet, dass das Volumenmischungsverhältnis azeotropes Gemisch (A) zu zugesetztem Ethylenglykol im Bereich von 1:2 bis 1:6 liegt.

In einer weiteren Ausführungsform 3 ist das Verfahren gemäß dem Aspekt dadurch gekennzeichnet, dass dem azeotropen Gemisch (A) Wasser zugesetzt wird.

In einer Ausführungsform 4 ist das Verfahren gemäß dem Aspekt und/oder der Ausführungsform 3 dadurch gekennzeichnet, dass das Volumenmischungsverhältnis azeotropes Gemisch (A) zu zugesetztem Wasser im Bereich von 1:2 bis 1:6 liegt.

In einer Ausführungsform 5 ist das Verfahren gemäß dem Aspekt und/oder einem der Ausführungsformen dadurch gekennzeichnet, dass ein weiterer Bestandteil des azeotropen Gemisches (A) Wasser ist.

In einer Ausführungsform 6 ist das Verfahren gemäß dem Aspekt und/oder einem der Ausführungsformen dadurch gekennzeichnet, dass weitere Bestandteile des azeotropen Gemisches (A) ausgewählt sind aus einer Gruppe bestehend aus Wasser, einem C1, einem C2, einem C4, einem C5, einem C6-Alkohol, oder Mischung daraus, umfassen.

In einer Ausführungsform 7 ist das Verfahren gemäß dem Aspekt und/oder einem der Ausführungsformen dadurch gekennzeichnet, dass weitere Bestandteile des azeotropen Gemisches (A) Syntheserückstände einer Synthese sind, in der das azeotropen Gemisches (A) als Lösungsmittel verwendet wurde.

In einer Ausführungsform 8 ist das Verfahren gemäß dem Aspekt und/oder einem der Ausführungsformen dadurch gekennzeichnet, dass das mechanische Entfernen der Toluol-reiche Phase (B) von der 1-Propanol-reiche Phase (C) durch eine flüssig-flüssig Phasentrennung erfolgt.

In einer Ausführungsform 9 ist das Verfahren gemäß dem Aspekt und/oder einem der Ausführungsformen 1, 2, 5 bis 8 dadurch gekennzeichnet, dass das Verfahren zusätzlich die Schritte
(iii) Auftrennung der 1-Propanol-haltigen Phase (C) durch Abdestillation des 1-Propanol in eine oder mehrere 1-Propanol-Fraktionen und eine Ethylenglykol-reiche Phase (D); und
(iv) die so gewonnene Ethylenglykol-reiche Phase (D) wieder für das Erreichen einer Mischungslücke eines azeotropen Gemisches (A) in Schritt (i) verwendet wird,
umfasst.

In einer Ausführungsform 10 ist das Verfahren gemäß dem Aspekt und/oder einem der Ausführungsformen 3 bis 8 dadurch gekennzeichnet, dass das Verfahren zusätzlich die Schritte
(iii) Auftrennung der 1-Propanol haltigen Phase (C) durch Abdestillation des 1-Propanol in eine oder mehrere 1-Propanol-Fraktionen und eine Wasser-reiche Phase (D') durch Abdestillation des 1-Propanol, und
(iv) die so gewonnene Wasser-reiche Phase (D') wieder für das Erreichen einer Mischungslücke eines azeotropen Gemisches (A) in Schritt (i) verwendet wird,
umfasst.

In einer Ausführungsform 11 ist das Verfahren gemäß Ausführungsform 10 oder 11 dadurch gekennzeichnet, dass die Reinheit des abdestillierten 1-Propanol 65% oder mehr beträgt.

In einer Ausführungsform 12 ist das Verfahren gemäß Aspekt 1 und/oder einer der Ausführungsformen 1 bis 11 weiterhin dadurch gekennzeichnet, dass es den Schritt bestimmen den Anteil an Toluol und 1-Propanil eines azeotropen Gemisches (A) und kalkulieren der Menge an zuzugebenden Wasser oder Ethylenglykol durch Abgleich mit einem T-x-Phasendiagramms eines ternären Systems aus 1-Propanol, Toluol und Wasser oder eines ternären Systems aus 1-Propanol, Toluol und Ethylenglykol vor Zugabe von Wasser oder Ethylenglykol gemäß Schritt (i) umfasst.

In einer Ausführungsform 13 ist das Verfahren gemäß Aspekt 1 und/oder einer der Ausführungsformen 1 bis 12 weiterhin dadurch gekennzeichnet, dass Schritt (i) bei einer Temperatur von 0°C bis 30°C durchgeführt wird.

In einer Ausführungsform 14 ist das Verfahren gemäß Aspekt 1 und/oder einer der Ausführungsformen 1 bis 13 weiterhin dadurch gekennzeichnet, dass Schritt (ii) bei einer Temperatur von 0°C bis 30°C durchgeführt wird.

Die Aufgabe ein azeotropes Gemisch (A) aus 1-Propanol und Toluol, optional ein ternäres, azeotropes Gemisch aus Wasser, 1-Propanol und Toluol, in eine 1-Propanol-reiche und eine Toluol-reiche Phase aufzutrennen wird erfindungsgemäß dadurch gelöst, dass eine überraschend gefundene Mischungslücke in dem azeotropen Gemisch (A) durch Zugabe von Ethylenglykol oder durch Zugabe von Wasser erreicht wird. Ein azeotropes Gemisch (A) entsteht häufig bei der Synthese von Stoffen und kann optional noch weitere Nebenprodukte aus dem vorangegangenen Syntheseschritt enthalten. Durch Einsatz mechanisch wirkender Flüssig-Flüssig-Trennapparate kann das kanzerogenen dabei durch das entstehende heterogenen Gemische von der 1-Propanol-haltigen Phase abgetrennt werden. Durch die mechanische Flüssig-Flüssig-Trennung des kanzerogenen Toluols von der 1-Propanol-haltigen Phase kann das 1-Propanol optional anschließend durch einfache Destillation aus der wässrigen/Ethylenglykolreichen Phase abgetrennt werden und das so erhaltene Wasser bzw. Ethylenglykol kann wieder in den Kreislauf zurückgeführt werden und erneut als Trennmittel verwendet werden. Das Toluol kann aufgrund der hohen Reinheit z. B. für weitere Syntheseschritte verwendet werden.

Das erfindungsgemäße Verfahren bietet eine kostengünstige abfall- und energieeffiziente Lösung zur Rückgewinnung von Toluol und/oder 1-Propanol aus einem Prozessdestillatstrom, ohne auf aufwendige Destillationsschritte zurückgreifen zu müssen.

Die Vorliegende Erfindung bezieht sich auf ein Verfahren zum Auftrennen eines azeotropen Gemisches (A), bevorzugt das Abtrennen von Toluol aus einem azeotropen Gemisch (A) umfassend Toluol und n-Propanol (1-Propanol) gekennzeichnet dadurch, dass durch Zugabe von Ethylenglykol und/oder Wasser eine Mischungslücke erreicht wird und Toluol unter Verwendung einer flüssig-flüssig Extraktion abgetrennt wird.

Besonders bevorzugt umfasst das erfindungsgemäße Verfahren nicht nur die Abtrennung von Toluol von 1-Propanol, sondern ermöglicht es auch einen Großteil des zur Auftrennung des azeotropen Gemisches zugesetzten Lösungsmittel (Wasser oder Ethylenglykol) wieder in den Auftrennungsprozess zurückzuführen. So wird eine weitere Abfallreduzierung ermöglicht.

Gegenüber dem Verfahren in CN106336337 ermöglicht das erfindungsgemäße Verfahren eine bessere Rückgewinnung und auf den Einsatz von Kolonnen und Vakuumpumpe kann hier ebenfalls verzichtet werden.

Gegenüber dem Verfahren aus DD 000000225049 werden hier nicht nur Spuren von Toluol aus einem azeotropen Gemisch entfernt, sondern Toluol wird aus einem azeotropen Gemisch mit einem sehr hohen Anteil an Toluol entfernt. Zudem bezieht sich die Offenbarung auf ein anderes azeotropes Gemisch.

### Beschreibung der Zeichnungen

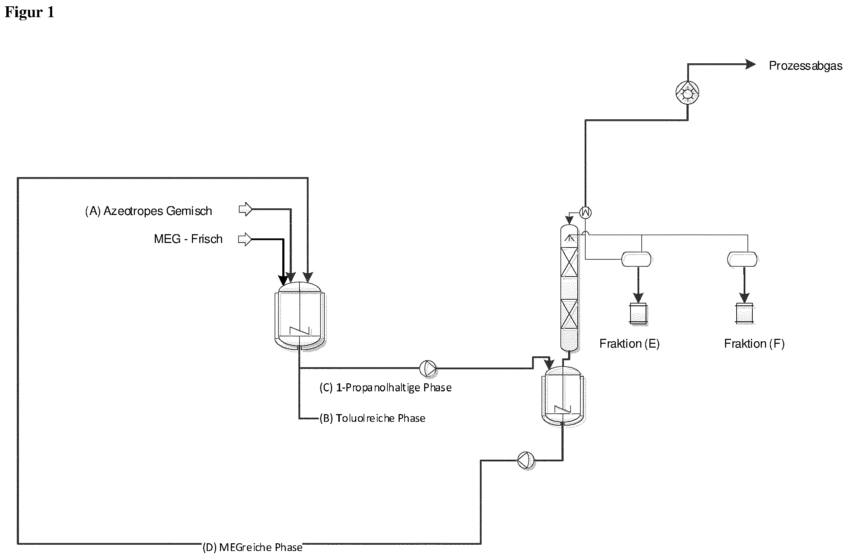

Figur 1 zeigt eine schematische Abtrennungsaufbau der geeignet ist, einen Strom eines azeotropen Gemisches (A) aus Toluol und n-Propanol durch Zugabe von Ethylenglykol (MEG) in eine obere Toluol-Phase (B) und eine untere Ethylenglykol- und 1-Propanol-reiche Phase (C) aufzutrennen. Aus Phase (C) kann dann durch (fragmentierte) Destillation 1-Propanol-reiche Fraktionen (hier (E) und (F)) abgetrennt werden. Die so gewonnene Ethylenglykol-reiche Phase (D) kann wieder für das Erreichen einer Mischungslücke eines azeotropen Gemisches (A) in Schritt (i) verwendet werden. Je nach Reinheit des fragmentiert destillierten 1-Propanols kann dieses ebenfalls für weitere Synthesen verwendet werden.

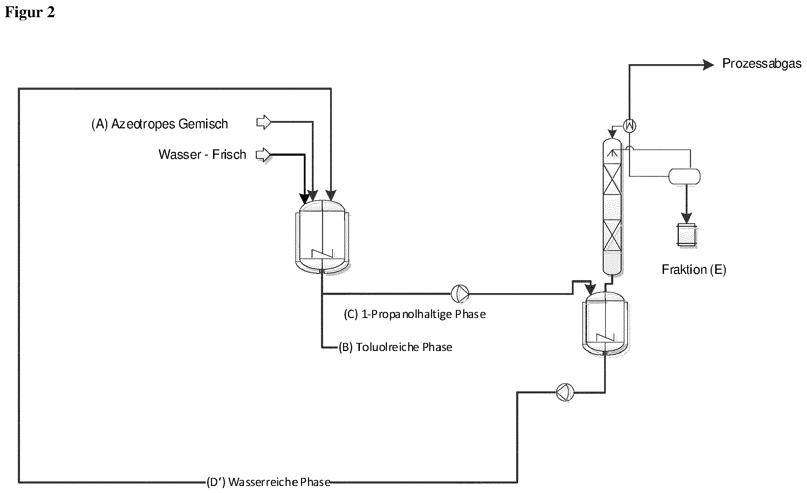

Figur 2 zeigt eine schematische Abtrennungsaufbau der geeignet ist, einen Strom eines azeotropen Gemisches (A) aus Toluol und n-Propanol durch Zugabe von Wasser in eine obere Toluol-Phase (B) und eine untere Wasser- und 1-Propanol-reiche Phase (C) aufzutrennen. Aus Phase (C) kann dann durch (fragmentierte) Destillation 1-Propanol-reiche Fraktionen (hier (E) und (F)) abgetrennt werden. Die so gewonnene Wasser-reiche Phase (D') kann wieder für das Erreichen einer Mischungslücke eines azeotropen Gemisches (A) in Schritt (i) verwendet werden. Je nach Reinheit des fragmentiert destillierten 1-Propanols kann dieses ebenfalls für weitere Synthesen verwendet werden.

### Beschreibung der bevorzugten Ausführungsformen

Wenn nicht anders angegeben sind %-Angaben in Gewichtsprozenten (% (w/w)) angegeben. Die in den hierin verwendeten gaschromatographischen Analysen ermittelten Flächenprozente werden 1:1 als Gewichtsprozente (% (w/w)) angegeben.

Der Fachmann versteht, dass die Summe aller Gewichtsprozente (% (w/w)) aller Komponenten bezogen auf eine Phase / eine Lösung / eine Mischung / ein Gemisch oder einer anderen Kombination von mindestens zwei Komponenten immer 100% (w/w) ergeben muss.

Die Gaschromatographische Analyse der Anteile in einem azeotropen Gemisch (A) und den einzelnen Fraktionen des erfindungsgemäßen Auftrennungsprozesses können mittels eines GC WLD (Wärmeleitfähigkeitsdetektor) Gaschromatographen bestimmt werden (z. B. Gerät: HP6890 PLUS, Säule CP-SIL-5CB 50 m × 0,53 mm x 5,00 µm (Chrompack).

Die Vorliegende Erfindung bezieht sich auf ein Verfahren zum Auftrennen eines azeotropen Gemisches umfassend Toluol und n-Propan (1-Propanol), bevorzugt Abtrennen von Toluol aus einem azeotropen Gemisch umfassend Toluol und n-Propan (1-Propanol), gekennzeichnet dadurch, dass durch Zugabe von Wasser und/oder Monoethylenglykol (Ethylenglykol) eine Mischungslücke erreicht wird und Toluol von 1-Propanol unter Verwendung einer flüssig-flüssig Extraktion getrennt wird.

Insbesondere auf ein Verfahren zur Auftrennung eines azeotropen Gemisches (A) umfassend Toluol und n-Propanol, bevorzugt der Abtrennung von Toluol aus einem azeotropen Gemisch (A), wobei der Anteil an Toluol in dem Gemisch im Bereich von 55% (w/w) bis 80% (w/w) bezogen auf das Gesamtgewicht des azeotropen Gemisches (A) liegt und der Anteil an n-Propanol im Bereich von 20% (w/w) bis 45% (w/w) bezogen auf das Gesamtgewicht des azeotropen Gemisches (A) liegt und der Anteil von weiteren Bestandteile des azeotropen Gemisches (A) im Bereich von 0% (w/w) bis 10% (w/w) bezogen auf das Gesamtgewicht des azeotropen Gemisches (A) liegt, wobei die Summe aus dem Toluol-Anteil, dem n-Propanol-Anteil und dem Anteil von weiteren Bestandteilen zusammen immer 100 ergeben müssen, dadurch gekennzeichnet, dass
(v) dem azeotropen Gemisch (A) Ethylenglycol oder Wasser zugesetzt wird, bis eine Mischungslücke erreicht ist, und
(vi) die entstehende Toluol-reiche Phase (B) von der 1-Propanol-haltigen Phase (C) mechanisch entfernt wird.

Toluol-reiche Phase (B) im Sinne der vorliegenden Erfindung bedeutet eine Phase, in der der Toluol Gehalt mindestens 85% (w/w), mehr bevorzugt mindestens 90% (w/w), noch mehr bevorzugt mindestens 95% (w/w) beträgt und der 1-Propanol Gehalt maximal 3,5% (w/w), mehr bevorzugt maximal 3% (w/w), noch mehr bevorzugt maximal 2,5% (w/w), wie z. B. 0,75% (w/w) ±0,05% (w/w), beträgt. Optional können weitere Bestandteile des azeotropen Gemisches (A) bzw. das zum Erreichen einer Mischungslücke des azeotropen Gemisches (A) zugegebene Lösungsmittel (Wasser oder Ethylenglykol) in Phase (B) vorliegen, falls der Anteil an Toluol und 1-Propanol nicht bereits 100% (w/w) ergibt.

1-Propanol-haltige Phase (C) im Sinne der vorliegenden Erfindung bedeutet eine Phase, in der der Toluolgehalt maximal 15% (w/w), mehr bevorzugt maximal 10% (w/w), noch mehr bevorzugt maximal 5% (w/w) beträgt, ganz besonders bevorzugt ist Toluol nicht in der Phase (C) vorhanden / bzw. nicht mehr mittels GC WLD nachweisbar; der Anteil an einem eine Mischungslücke des azeotropen Gemisches (A) Lösungsmittels (Wasser oder Ethylenglykol) mindestens 70% (w/w), mehr bevorzugt mindestens 80% (w/w), noch mehr bevorzugt mindestens 85% (w/w), wie etwa 95% (w/w), beträgt; und der Anteil von 1-Propanol mindestens 2,5 % (w/w) mehr bevorzugt mindestens 3% (w/w), noch mehr bevorzugt mindestens 4% (w/w), wie z. B. 5,5% (w/w) ± 0,5% (w/w) oder 4,5% (w/w) ± 0,5% (w/w), beträgt. Optional können weitere Bestandteile des azeotropen Gemisches (A) vorliegen, falls der Anteil an 1-Propanol und dem zum Erreichen einer Mischungslücke des azeotropen Gemisches (A) zugegebene Lösungsmittel (Wasser oder Ethylenglykol) nicht bereits 100% (w/w) ergibt.

In einer bevorzugten Ausführungsform ist die Reinheit des abgetrennten Toluols mindestens 95%, mehr bevorzugt mindestens 97%, noch mehr bevorzugt mindestens 98%, wie z. B. 98,5% und mehr oder 99% und mehr.

Dieses Verfahren zum Auftrennen von 1-Propanol und Toluol nach einem Syntheseschritt kann in jeder Synthese genutzt werden, in der die beiden Lösungsmittel eingesetzt werden. Um Beispiel kann ein solcher Lösungsmitteltausch bei der Synthese von agrochemischen Substanzen wie einigen Ketoenolen genutzt werden.

Der Fachmann wird verstehen, dass sich noch Nebenprodukte und/oder Rückstände aus einer vorausgegangenen Synthese als weitere Bestandteil in einem azeotropen Gemisch aus 1-Propanol und Toluol befinden können.

Bevorzugt wird ein Syntheseprodukt vor der Auftrennung des azeotropen Gemisches (A) aus diesem entfernt. Bevorzugt geschieht die Entfernung durch thermische Trennung (z. B. Destillation). Dabei kann das Syntheseprodukt abdestilliert werden oder das azeotrope Gemisch (A) wird abdestilliert.

Typische Nebenprodukte und/oder Rückstände, die sich in dem azeotropen Gemisch als weitere Bestandteile befinden können, sind z. B. Wasser, andere protische Lösungsmittel wie Methanol, Ethanol, Butanol, Pentanol oder Hexanol, Carbonsäuren (z. B. Ameisensäure oder Essigsäure) oder deren Alkali oder Erdalkalisalze, primäre oder sekundäre Amide (z. B. Formamid, Acetamid, Harnstoff, Phthalimid, N,N-Dimethalformamid (DMF)), ein oder mehrere Mineralsäuren (z. B. Salzsäure, Schwefelsäure, Salpetersäure oder deren Alkali- oder Erdalkalisalze, optional mono- oder poly-chlorierte und/oder mono- oder poly-fluorierte Alkane (z. B. Ethan, Propan, Butan Pentan, Hexan, Cyclohexan, Chlorethan, Fluorethan, Chlorpropan, Fluorpropan nicht verbrauchte Edukte einer Synthese oder Nebenprodukte einer Synthese.

Der Fachmann ist in der Lage, je nach Synthese aus dem das azeotrope Gemisch (A) (in den Beispielen das "Prozessdestillat") die entsprechenden zu erwartenden Edukte und Nebenprodukte zu charakterisieren und nachzuweisen.

In einer bevorzugten Ausführungsform umfasst der Ausdruck "weitere Bestandteile" Wasser.

In einer bevorzugten Ausführungsform umfasst der Ausdruck "weitere Bestandteile" Wasser und einen Alkohol ausgewählt aus der Gruppe bestehend aus Methanol, Ethanol, Butanol, Pentanol oder Hexanol.

In einer bevorzugten Ausführungsform sind die Nebenprodukte und/oder Rückstände in einem azeotropen 1-Propanol/Toluol Gemisch (A) Wasser, HCl, Methanol und Chlorpropan.

### Anteil Toluol an (A)

In einer weiteren bevorzugten Ausführungsform beträgt der Anteil des Toluols an einem azeotropen Gemisch (A) zwischen 55% (w/w) und 80% (w/w), mehr bevorzugt zwischen 60% (w/w) und 80% (w/w), noch mehr bevorzugt zwischen 65% (w/w) und 75% (w/w).

### Anteil 1-Propanol an (A)

In einer weiteren bevorzugten Ausführungsform beträgt der Anteil des 1-Propanol an einem azeotropen Gemisch (A) zwischen 20% (w/w) und 45% (w/w), mehr bevorzugt zwischen 20% (w/w) und 35% (w/w), wie zwischen 20% (w/w) und 30% (w/w) oder zwischen 25% (w/w) und 35% (w/w).

In einer weiteren bevorzugten Ausführungsform beträgt der Anteil weitere Bestandteile in einem azeotropen Gemisch maximal 10% (w/w), mehr bevorzugt maximal 7% (w/w), noch mehr bevorzugt maximal 6% (w/w), besonders bevorzugt maximal 5% (w/w).

### Wasser

In einer weiteren bevorzugten Ausführungsform umfasst ein azeotropes Gemisch (A) neben Toluol und 1-Propanol noch Wasser. Der Fachmann weiß, dass es sich bei dem System Toluol, 1-Propanol und Wasser um ein ternäres azeotropes Gemisch handelt.

In einer weiteren bevorzugten Ausführungsform ist ein weiterer Bestandteil eines azeotropen Gemisches (A) Wasser und der Anteil des Wassers an dem azeotropen Gemisch (A) beträgt maximal 5% (w/w), mehr bevorzugt zwischen 2% (w/w) und 5% (w/w).

In einer weiteren bevorzugten Ausführungsform beträgt die Summe aller weiteren Bestandteile außer Wasser eines azeotropen Gemisches (A) maximal 1,5% (w/w), mehr bevorzugt maximal 1% (w/w).

### Methanol

In einer weiteren bevorzugten Ausführungsform umfasst ein azeotropes Gemisch (A) neben Toluol und 1-Propanol noch Methanol.

Bevorzugt beträgt der Anteil von Methanol an dem azeotropen Gemisch (A) maximal 1% (w/w), mehr bevorzugt maximal 0,75% (w/w), noch mehr bevorzugt maximal 0,5% (w/w).

In einer weiteren bevorzugten Ausführungsform umfasst ein azeotropes Gemisch (A) neben Toluol und 1-Propanol noch Wasser und Methanol.

Bevorzugt liegt der Anteil von Wasser an dem azeotropen Gemisch (A) zwischen 2% (w/w) und 5% (w/w) und der Anteil Methanol an dem azeotropen Gemisch (A) zwischen 0,1% (w/w) und 0,5% (w/w).

### Volumenmischungsverhältnis Ethylenglykol zu (A)

In einer weiteren bevorzugten Ausführungsform liegt das Volumenmischungsverhältnis azeotropes Gemisch (A) zu Ethylenglykol in Schritt (i) zwischen 1:2 und 1:6 wie zwischen 1:2 und 1:5 oder mehr bevorzugt zwischen 1:2 und 1:4, noch mehr bevorzugt zwischen 1:2,5 und 1:3,5 (z. B. bei etwa 1:3).

In einer weiteren bevorzugten Ausführungsform liegt das Volumenmischungsverhältnis azeotropes Gemisch (A) zu Ethylenglykol in Schritt (i) zwischen 1 zwischen 1:2,5 und 1:3,5 und der Anteil des Toluols an dem azeotropen Gemisch (A) liegt zwischen 60% (w/w) und 80% (w/w) und der Anteil des 1-Propanols an dem azeotropen Gemisch (A) liegt zwischen 20% (w/w) und 35% (w/w).

In einer weiteren bevorzugten Ausführungsform liegt das Volumenmischungsverhältnis azeotropes Gemisch (A) zu Ethylenglykol in Schritt (i) liegt zwischen 1:2,5 und 1:3,5 und der Anteil des Toluols an dem azeotropen Gemisch (A) liegt zwischen 65% (w/w) und 75% (w/w), der Anteil des 1-Propanols an dem azeotropen Gemisch (A) liegt zwischen 20% (w/w) und 30% (w/w).

### Volumenmischverhältnis Wasser zu (A)

In einer weiteren bevorzugten Ausführungsform liegt das Volumenmischungsverhältnis azeotropes Gemisch (A) zu Wasser in Schritt (i) zwischen 1:2 und 1:6 wie zwischen 1:2 und 1:5 oder mehr bevorzugt zwischen 1:2 und 1:4, noch mehr bevorzugt zwischen 1:2,5 und 1:3,5 (z. B. bei etwa 1:3).

In einer weiteren bevorzugten Ausführungsform liegt das Volumenmischungsverhältnis azeotropes Gemisch (A) zu Wasser in Schritt (i) zwischen 1 zwischen 1:2,5 und 1:3,5 und der Anteil des Toluols an dem azeotropen Gemisch (A) liegt zwischen 60% (w/w) und 80% (w/w) und der Anteil des 1-Propanols an dem azeotropen Gemisch (A) liegt zwischen 20% (w/w) und 35% (w/w).

In einer weiteren bevorzugten Ausführungsform liegt das Volumenmischungsverhältnis azeotropes Gemisch (A) zu Ethylenglykol in Schritt (i) liegt zwischen 1:2,5 und 1:3,5 und der Anteil des Toluols an dem azeotropen Gemisch (A) liegt zwischen 65% (w/w) und 75% (w/w), der Anteil des 1-Propanols an dem azeotropen Gemisch (A) liegt zwischen 20% (w/w) und 30% (w/w).

### Massenverhältnis Ethylenglykol zu (A)

In einer weiteren bevorzugten Ausführungsform liegt das Massenmischungsverhältnis azeotropes Gemisch (A) zu Ethylenglykol in Schritt (i) zwischen 1:2,6 und 1:7,7, zwischen 1:2,6 und 1:6,4 zwischen 1:2,6 und 1:5,1, mehr bevorzugt zwischen 1:3,2 und 1:4,5.

In einer weiteren bevorzugten Ausführungsform liegt das Massenmischungsverhältnis azeotropes Gemisch (A) zu Ethylenglykol in Schritt (i) zwischen 1:3,2 und 1:4,5 und der Anteil des Toluols an dem azeotropen Gemisch (A) liegt zwischen 60% (w/w) und 80% (w/w) und der Anteil des 1-Propanols an dem azeotropen Gemisch (A) liegt zwischen 20% (w/w) und 35% (w/w).

In einer weiteren bevorzugten Ausführungsform liegt das Massenmischungsverhältnis azeotropes Gemisch (A) zu Ethylenglykol in Schritt (i) liegt zwischen 1:3,2 und 1:4,5 und der Anteil des Toluols an dem azeotropen Gemisch (A) liegt zwischen 65% (w/w) und 75% (w/w), der Anteil des 1-Propanols an dem azeotropen Gemisch (A) liegt zwischen 20% (w/w) und 30% (w/w).

### Massenverhältnis Wasser zu (A)

In einer weiteren bevorzugten Ausführungsform liegt das Massenmischungsverhältnis azeotropes Gemisch (A) zu Wasser in Schritt (i) zwischen 1:2 und 1:6, bevorzugt zwischen 1:2 und 1:5, mehr bevorzugt zwischen 1:2 und 1:4, noch mehr bevorzugt zwischen 1:2,5 und 1:3,5 (z. B. bei 1:3).

In einer weiteren bevorzugten Ausführungsform liegt das Massenmischungsverhältnis azeotropes Gemisch (A) zu Wasser in Schritt (i) zwischen 1:2,5 und 1:4,5 und der Anteil des Toluols an dem azeotropen Gemisch (A) liegt zwischen 60% (w/w) und 80% (w/w) und der Anteil des 1-Propanols an dem azeotropen Gemisch (A) liegt zwischen 20% (w/w) und 35% (w/w).

In einer weiteren bevorzugten Ausführungsform liegt das Massenmischungsverhältnis azeotropes Gemisch (A) zu Wasser in Schritt (i) liegt zwischen 1:2,5 und 1:4,5 und der Anteil des Toluols an dem azeotropen Gemisch (A) liegt zwischen 65% (w/w) und 75% (w/w), der Anteil des 1-Propanols an dem azeotropen Gemisch (A) liegt zwischen 25% (w/w) und 35% (w/w).

### Temperatur Schritt (i) und Schritt (ii)

In einer weiteren bevorzugten Ausführungsform wird Schritt (i) bei einer Temperatur zwischen 0°C und 30°C durchgeführt, mehr bevorzugt zwischen 4°C und 28°C, wie bei Raumtemperatur.

In einer weiteren bevorzugten Ausführungsform wird Schritt (ii) bei einer Temperatur zwischen 0°C und 30°C durchgeführt, mehr bevorzugt zwischen 4°C und 28°C, wie bei Raumtemperatur.

### Druck

In einer weiteren bevorzugten Ausführungsform wird Schritt (i) bevorzugt im Bereich des Normaldrucks (1,01325 bar) durchgeführt wie bei einem Druck im Bereich von 0,9 bar bis 1,1 bar, oder 0,95 bar bis 1,05 bar.

In einer weiteren bevorzugten Ausführungsform wird Schritt (ii) bevorzugt im Bereich des Normaldrucks (1,01325 bar) durchgeführt wie bei einem Druck im Bereich von 0,9 bar bis 1,1 bar, oder 0,95 bar bis 1,05 bar.

### Destillation

1-Propanol kann von der 1-Propanol-haltigen Phase (C) mit Standarddestillationsverfahren abdestilliert werden. Das Abdestillieren von 1-Propanol kann auch fraktioniert erfolgen. Gewöhnlich kann bei Normaldruck destilliert werden, jedoch kann die Destillation auch bei vermindertem Druck erfolgen.

Die (fragmentierte) Destillation einer 1-Propanol-haltigen Phase (C) erfolgt gewöhnlich bei Standarddruck, insbesondere, wenn das zugegebene Lösungsmittel zum Erreichen der Mischungslücke des azeotropem Gemisches Wasser ist. Die (fragmentierte Destillation kann jedoch auch in einer weiteren, bevorzugten Ausführungsform bei vermindertem Druck, wie beispielsweise bei einem Druck von 2 mbar bei 100 mbar, oder bei einem Druck von 2 mbar bis 50 mbar, erfolgen, insbesondere, wenn das Lösungsmittel zum Erreichen der Mischungslücke des azeotropem Gemisches Ethylenglykol ist.

Die Destillation erfolgt gewöhnlich in einem Bereich von 60°C bis 110°C wie etwa im Bereich von 85°C bis 100°C, wie 93°C bis 100°C.

### Beispiel

### Beispiel 1

Wie in Figur 1 gezeigt, wurde ein Prozessdestillat (azeotropes Gemisch (A) gemäß Tabelle 1) zur Auftrennung verwendet. In einem Reaktor wurden Ethylenglykol und das azeotrope Gemisch (A) gegeben (6 l Ethylenglykol/ 2 l azeotropen Gemisches (A) und 15 Minuten bei Raumtemperatur gerührt, wonach sich das nun heterogene Zweiphasengemisch absetzen konnte. Eine Toluol-reiche Phase wurde durch die Flüssig-Flüssig-Extraktion als obere Phase (B) in der nun heterogenen Mischung erzeugt. Die obere Phase (B) bestand aus über 99% reinem Toluol mit geringen Verunreinigungen durch 1-PrOH. Die untere, 1-Propanol-haltige Phase (C) bestand aus einer Mischung von n-Propanol, Methanol, Wasser und Ethylenglykol. Diese Mischung wurde in Reaktor B übertragen und durch Destillation bei 50 mbar und bei 95°C weiter gereinigt. Der verbleibende Inhalt (Ethylenglykol-reiche Phase D) bestand aus über 99% reinem Ethylenglykol und wurde abgekühlt und in den Reaktor zur Erneuten Aufarbeitung des azeotropen Gemisches (A) zurückgeführt. Für die erneute Aufarbeitung wurde frisches Ethylenglykol lediglich in der Menge hinzugefügt, um die Verluste durch das Abdestillieren des 1-Propanol aus dem Ethylenglykol auszugleichen.

Der Ablauf ist schematisch in Figur 1 dargestellt.

Fraktion (E) und Fraktion (F) wurden in diesem Beispiel nicht weiterverwendet und darum fachgerecht entsorgt. Zumindest Fraktion (F) kann jedoch weiter aufgearbeitet/ und erneut oder direkt wieder für Reaktionen verwendet werden.

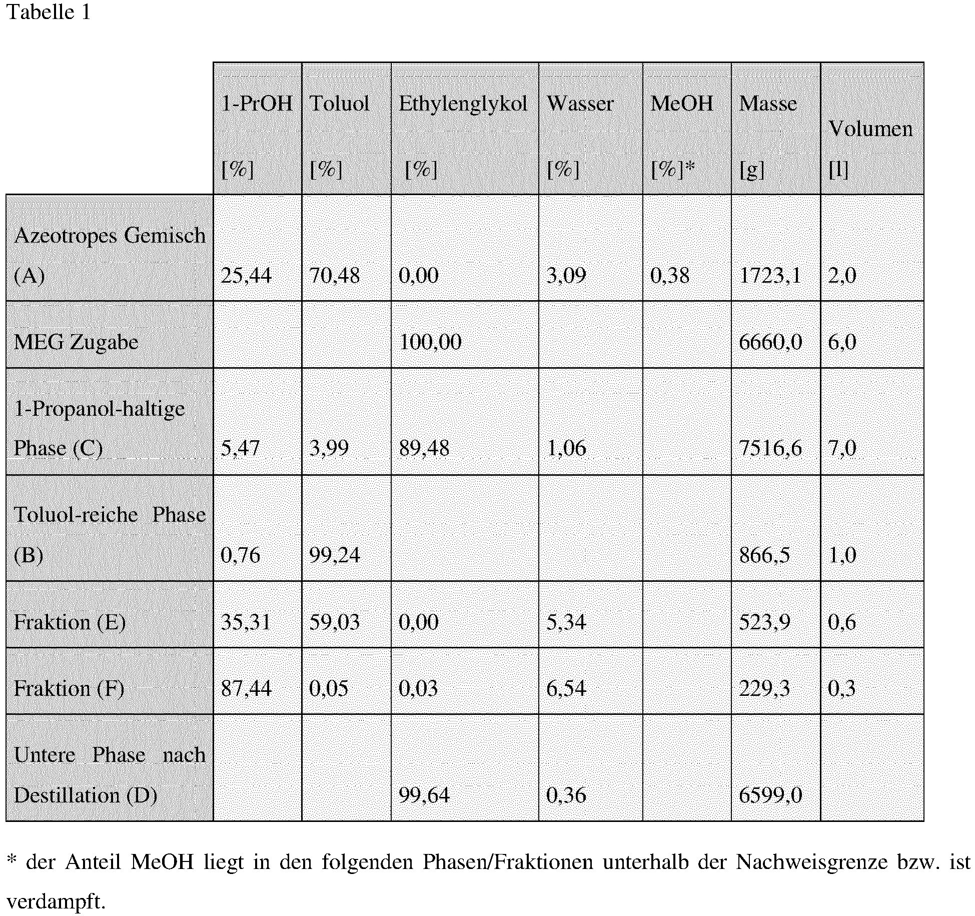

### Beispiel 2

Wie in Figur 2 gezeigt, wurde ein Prozessdestillat (azeotropes Gemisch (A) gemäß Tabelle 2) zur Auftrennung verwendet. In einem Reaktor wurden Wasser und das azeotrope Gemisch (A) gegeben (2,55 g Wasser/g Prozessdestillat, das 4,44 g Wasser enthielt) gegeben und das Gemisch 15 Minuten bei Raumtemperatur gerührt, wonach sich das nun heterogene Zweiphasengemisch absetzen konnte. Die Toluol-reiche Phase wurde durch die Flüssig-Flüssig-Extraktion als obere Phase (B) in der nun heterogenen Mischung erzeugt. Die untere Phase besteht aus einer Mischung von n-Propanol und Wasser. Diese Mischung wurde dann in Reaktor B geleitet und dort durch Destillation bei Normaldruck und bei 95°C weiter gereinigt. Der verbleibende Inhalt (Wasser-reiche Phase D') wurde abgekühlt und in den Reaktor zur Erneuten Aufarbeitung des azeotropen Gemisches (A) zurückgeführt. Für die erneute Aufarbeitung wurde frisches Wasser hinzugefügt, um die Verluste durch das Abdestillieren des 1-Propanol aus dem Wasser auszugleichen.

Der Ablauf ist schematisch in Figur 2 dargestellt.

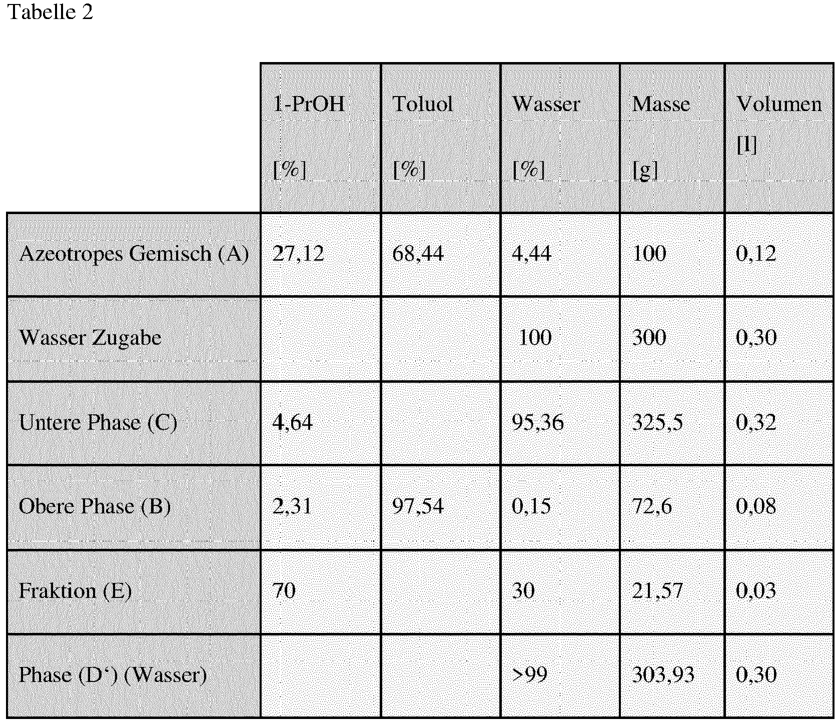

### Gaschromatographie

Die Verhältnisse der Einzelnen Komponenten wurden mit Hilfe von GC WLD bestimmt.

Gerät: HP6890 PLUS; Säule CP-SIL-5CB 50 m x 0,53 mm x 5,00 µm (Chrompack); 60°C, 10°C/min bis 100°C, dann 20°C/min bis 300°C; Trägergas: Helium

## Patentansprüche

1. Verfahren zur Auftrennung eines azeotropen Gemisches (A) umfassend Toluol und n-Propanol, wobei der Anteil an Toluol in dem Gemisch im Bereich von 55% (w/w) bis 80% (w/w) bezogen auf das Gesamtgewicht des azeotropen Gemisches (A) liegt und der Anteil an n-Propanol im Bereich von 20% (w/w) bis 45% (w/w) bezogen auf das Gesamtgewicht des azeotropen Gemisches (A) liegt und der Anteil von weiteren Bestandteile des azeotropen Gemisches (A) im Bereich von 0% (w/w) bis 10% (w/w) bezogen auf das Gesamtgewicht des azeotropen Gemisches (A) liegt, wobei die Summe aus dem Toluol-Anteil, dem n-Propanol-Anteil und dem Anteil von weiteren Bestandteilen zusammen immer 100 ergeben müssen, **dadurch gekennzeichnet, dass**
(i) dem azeotropen Gemisch (A) Ethylenglycol oder Wasser zugesetzt wird, bis eine Mischungslücke erreicht ist, und
(ii) die entstehende Toluol-reiche Phase (B) von der 1-Propanol-haltigen Phase (C) mechanisch entfernt wird.

2. Verfahren gemäß Anspruch 1, **dadurch gekennzeichnet, dass** dem azeotropen Gemisch (A) in Schritt (i) Ethylenglykol zugesetzt wird.

3. Verfahren gemäß Anspruch 2, **dadurch gekennzeichnet, dass** das Volumenmischungsverhältnis azeotropes Gemisch (A) zu zugesetztem Ethylenglykol im Bereich von 1:2 bis 1:6 liegt.

4. Verfahren gemäß Anspruch 1, **dadurch gekennzeichnet, dass** dem azeotropen Gemisch (A) Wasser zugesetzt wird.

5. Verfahren gemäß Anspruch 4, **dadurch gekennzeichnet, dass** das Volumenmischungsverhältnis azeotropes Gemisch (A) zu zugesetztem Wasser im Bereich von 1:2 bis 1:6 liegt.

6. Verfahren gemäß einem der vorherigen Ansprüche, wobei ein weiterer Bestandteil des azeotropen Gemisches (A) Wasser ist.

7. Verfahren gemäß einem der vorherigen Ansprüche, wobei weitere Bestandteile des azeotropen Gemisches (A) ausgewählt sind aus einer Gruppe bestehend aus Wasser, einem C1, einem C2, einem C4, einem C5, einem C6-Alkohol, oder Mischung daraus, umfassen.

8. Verfahren gemäß einem der vorherigen Ansprüche, wobei weitere Bestandteile des azeotropen Gemisches (A) Syntheserückstände einer Synthese sind, in der das azeotropen Gemisches (A) als Lösungsmittel verwendet wurde.

9. Verfahren gemäß einem der vorherigen Ansprüche, wobei das mechanische Entfernen der Toluol-reiche Phase (B) von der 1-Propanol-haltigen Phase (C) durch eine flüssig-flüssig Phasentrennung erfolgt.

10. Verfahren gemäß Anspruch 1 bis 3 oder einem der Ansprüche 6 bis 9, wobei das Verfahren zusätzlich die Schritte
(iii) Auftrennung der 1-Propanol-haltigen Phase (C) durch Abdestillation des 1-Propanol in eine oder mehrere 1-Propanol-Fraktionen und eine Ethylenglykol-reiche Phase (D); und
(iv) die so gewonnene Ethylenglykol-reiche Phase (D) wieder für das Erreichen einer Mischungslücke eines azeotropen Gemisches (A) in Schritt (i) verwendet wird,
umfasst.

11. Verfahren gemäß Anspruch 1 oder einem der Ansprüche 4 bis 9, wobei das Verfahren zusätzlich die Schritte
(iii) Auftrennung der 1-Propanol-haltigen Phase (C) durch Abdestillation des 1-Propanol in eine oder mehrere 1-Propanol-Fraktionen und eine Wasser-reiche Phase (D), und
(iv) die so gewonnene Wasser-reiche Phase (D') wieder für das Erreichen einer Mischungslücke eines azeotropen Gemisches (A) in Schritt (i) verwendet wird
umfasst.

12. Verfahren gemäß Anspruch 10 oder 11, wobei die Reinheit des abdestillierten 1-Propanol 65% oder mehr beträgt.

13. Verfahren gemäß einem der vorherigen Schritte umfassend den Schritt Bestimmen des Anteils an Toluol und 1-Propanil eines azeotropen Gemisches (A) und kalkulieren der Menge an zuzugebendem Wasser oder Ethylenglykol durch Abgleich mit einem T-x-Phasendiagramms eines ternären Systems aus 1-Propanol, Toluol und Wasser oder eines ternären Systems aus 1-Propanol, Toluol und Ethylenglykol, vor Zugabe von Wasser oder Ethylenglykol gemäß Schritt (i).

14. Verfahren gemäß einem der vorherigen Ansprüche weiterhin **dadurch gekennzeichnet, dass** Schritt (i) bei einer Temperatur von 0°C bis 30°C durchgeführt wird.

15. Verfahren gemäß einem der vorherigen Ansprüche weiterhin **dadurch gekennzeichnet, dass** Schritt (ii) bei einer Temperatur von 0°C bis 30°C durchgeführt wird.
